# EUROPEAN PATENT APPLICATION

(11) **EP 1 980 623 A1**
(43) Date of publication of application: **15.10.2008**
(21) Application number: 06807963.1
(22) Date of filing: 30.10.2006
(51) Int. Cl.: C12Q 1/68, G01N 33/50, A61P 13/12

(54) **A METHOD FOR IDENTIFYING A RENAL FIBROSIS PROCESS, USE OF SNAIL-ACTIVITY-INHIBITING COMPOUNDS IN THE PRODUCTION OF PHARMACEUTICAL COMPOSITIONS, METHOD FOR IDENTIFYING SAID INHIBITING COMPOUNDS, SAID PHARMACEUTICAL COMPOSITIONS AND APPLICATIONS THEREOF**

(30) Priority: 19.01.2006 ES 200600119
(71) Applicant: Consejo Superior de Investigaciones Cientificas (CSIC), 28006 Madrid (ES); Universidad Miguel Hernandez de Elche, 03202 Elche (Alicante) (ES)
(72) Inventor: NIETO TOLEDANO, María, Angela, E-03550 San Juan (Alicante) (ES); BOUTET, Agnès, E-03550 San Juan (Alicante) (ES); ALVAREZ DE FRUTOS, Cristina, E-03550 San Juan (Alicante) (ES)
(74) Representative: Pons Arino, Angel
(86) International application number: PCT/ES2006/070165
(87) International publication number: WO 2007/082967

(57) **Abstract**

Snail is a protein directly involved in the etiopathogeny of renal fibrosis as well as being a marker for this disease. Therefore, its identification may be used as a renal-fibrosis diagnosis. Also, furthermore, the Snail protein may be of great use in the identification of new drugs for the treatment of renal fibrosis, and its gene inhibition as a form of treatment.

## Description

### TECHNICAL FIELD OF THE INVENTION

This invention belongs to the field of biomedicine and, more specifically, to the application of biotechnological tools for the diagnosis and treatment of human diseases and, more specifically, of renal fibrosis.

### STATE OF THE ART

The renal epithelium originates from cells that suffer a mesenchyme-epithelium transition (MET). The reverse process, epithelium-mesenchyme transition (EMT) has been involved in the progression of epithelial tumours and in the fibrosis which ultimately leads to renal failure. Snail transcription factors induce both physiological and pathological EMTs by the repression of E-Cadherin transcription (amongst other targets) (Cano et al., 2000; Batlle et al., 2000; Bolos et al., 2003). It has been determined that Snail also suppresses the expression of kidney-specific cadherin, Cadherin 16 (Thompson et al., 1995), by repressing the transcription of the activator thereof, HNF-1beta (Hepatic nuclear factor-1 beta; Bai et al., 2002). This repression is active during early embryo development and it has been observed that the disappearance of Snail is concomitant with the appearance of HNF-1beta and, subsequently, of Cadherin 16, which are identified as signs of differentiation of the renal epithelium (Dressler, 2002). Snail activation in the mature kidney may be considered as a return to embryogenic properties and has been identified as sufficient to induce EMT and renal fibrosis in mice.

Progressive renal fibrosis is a devastating disease that triggers renal failure in patients suffering from various diseases, such as glomerulonephritis, IgA nephropathy, diabetes, renal damage induced by toxicity, urinary obstruction or deterioration of kidney transplants, amongst others (Liu, 2004; Kalluri and Neilson, 2003, Zeisberg and Kalluri, 2004; Vongwiwatana et al., 2005). It used to be believed that renal fibrosis arose from the activation of interstitial fibroblasts, but there are conclusive data which suggest that it also arises as a result of an EMT suffered by the renal tubules' epithelial cells (Iwano et al., 2002). Signs of tubular EMT have been observed in the kidneys of patients with renal fibrosis (Jinde et al., 2001; Rastaldi et al., 2002) and, in animal models, it has been calculated that about 36% of the fibroblast population arises from a local EMT of epithelial cells (Kalluri and Neilson, 2003).

On the other hand, Snail expression is concomitant with the EMT suffered in the kidney following the unilateral ureteral obstruction that causes renal fibrosis (Sato et al., 2003), although a cause-effect has not been established, since the cause of renal fibrosis was the ureteral obstruction. Furthermore, Snail expression is concomitant with the EMT suffered by mesothelial cells, which constitutes a mesothelial fibrosis (Yáñez-Mo et al., 2003), in the dialisates of patients subject to peritoneal dialysis and in culture cells treated with TGF-beta.

The use of BMP-7 as an inhibitory agent with the capacity to reverse renal fibrosis is patented. BMP-7 (bone morphogenic protein number 7) exerts this effect due to its capacity to inhibit TGF-beta, an agent that induces renal fibrosis and is known to induce Snail (reviewed in Barrallo-Gimeno and Nieto, 2005; United States Patent Application 20020173453, Method of treating renal injury). Both TGF-beta and BMP-7 are extracellular signalling molecules which initiate a complex cascade of events. If the induction of Snail is sufficient to reproduce renal fibrosis, the specific inhibition of Snail is expected to be a much more specific therapy than the inhibition of the entire TGF-beta signalling cascade.

Finally, if Snail activity in the mature kidney is sufficient to induce EMT and renal fibrosis, the presence of Snail could be considered to be a marker of renal fibrosis, the inhibition thereof could be considered as a form of anti-fibrotic therapy and Snail could be of great utility in identifying new anti-fibrotic drugs.

### DESCRIPTION OF THE INVENTION

### BRIEF DESCRIPTION

An object of this invention is a method of identifying a renal fibrosis process in humans, hereinafter renal fibrosis process identification method of the invention, based on the identification of the presence of Snail in a biological sample, which comprises the following steps:
a) identification of the presence of Snail, in a biological sample of renal origin, and
b) comparison of the presence of Snail observed in a) with the absence thereof in a control sample, where its presence is indicative of the existence of renal fibrosis.

A particular object of the invention is the identification method of the invention, wherein the identification of Snail of a) relates to the human forms of Snail1 (hSnail1, SEQ ID NO 5 and 6) and (hSnail2, SEQ ID NO 7 and 8), whether the identification is in the form of a gene transcript (mRNA) or the protein form of both genes.

Another object of this invention is a method of identifying and evaluating the activity of Snail protein inhibitory compounds which are useful to treat renal fibrosis, hereinafter compound identification method of this invention, which comprises the following steps:
a) Bringing into contact a biological system wherein there is a Snail expression that produces renal fibrosis with the candidate compound that is the object of this method, and incubation under suitable conditions,
b) determination of an indicative parameter of the renal fibrosis process, and
c) identification of a compound that inhibits Snail protein activity when a reduction of said renal fibrosis parameter is observed.

Another particular object of this invention is the identification method of the invention where the biological system of point a) is a transgenic animal where the expression of the Snail protein is inducible, in a constant or conditional manner, and where the expression thereof causes renal fibrosis. A particular embodiment is one wherein the transgenic animal is the transgenic mouse of this invention (Example 2, transgSnail1-ER mouse).

Another object of this invention is the use of a compound or agent that inhibits Snail protein activity, hereinafter use of a compound of this invention, in the preparation of drugs or pharmaceutical compositions for the treatment of renal fibrosis, preferably human.

Therefore, in another particular embodiment of the invention, said use of a compound is based on the fact that the inhibitory compound is a nucleic acid or polynucleotide which prevents or reduces the expression of the human Snail protein encoding gene and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

A particular embodiment of the invention is the use of an iRNA which preferably binds to the Snail mRNA gatgcacatccgaagccac (SEQ ID NO 9) fragment sequence or to another fragment that comprises the latter.

Another object of this invention is a pharmaceutical composition or a drug for the treatment of renal fibrosis, hereinafter pharmaceutical composition of this invention, which comprises a therapeutically effective quantity of a compound or agent that inhibits the Snail protein, jointly with, optionally, one or more pharmaceutically acceptable adjuvants and/or vehicles.

A particular embodiment of the invention is a pharmaceutical composition wherein the inhibitory compound is a nucleic acid or polynucleotide which prevents or reduces the expression of the human Snail protein encoding gene and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

Another object of this invention is the use of the pharmaceutical composition of the invention in a treatment method for a mammal, preferably a human being, suffering from renal fibrosis, hereinafter use of the pharmaceutical composition of this invention, which consists of administering said therapeutic composition that inhibits the fibrosis process.

A particular object of this invention is the use of the pharmaceutical composition of this invention wherein the renal fibrosis is caused by a disease, disorder or pathology which, for illustrative purposes, and without this limiting the scope of the invention, belongs to the following group: glomerulonephritis, IgA neuropathy, diabetes, renal damage induced by toxicity, urinary obstruction and deterioration of kidney transplants.

### DETAILED DESCRIPTION

This invention is based on the fact that the inventors have observed that Snail genes repress *in vivo* expression of cadherin-16 by indirectly repressing the gene transcription of the activator thereof, HNF1beta, in both cellular and animal models. In order to determine whether both Snail genes may repress cadherin-16 *in vivo,* their relative expression patterns were studied during embryo development in mice, when different EMT and MET processes take place that lead to the formation of the mature kidney (Example 1), in transgenic mice with inducible expression of the Snail genes (Example 2) and in samples from patients with renal fibrosis.

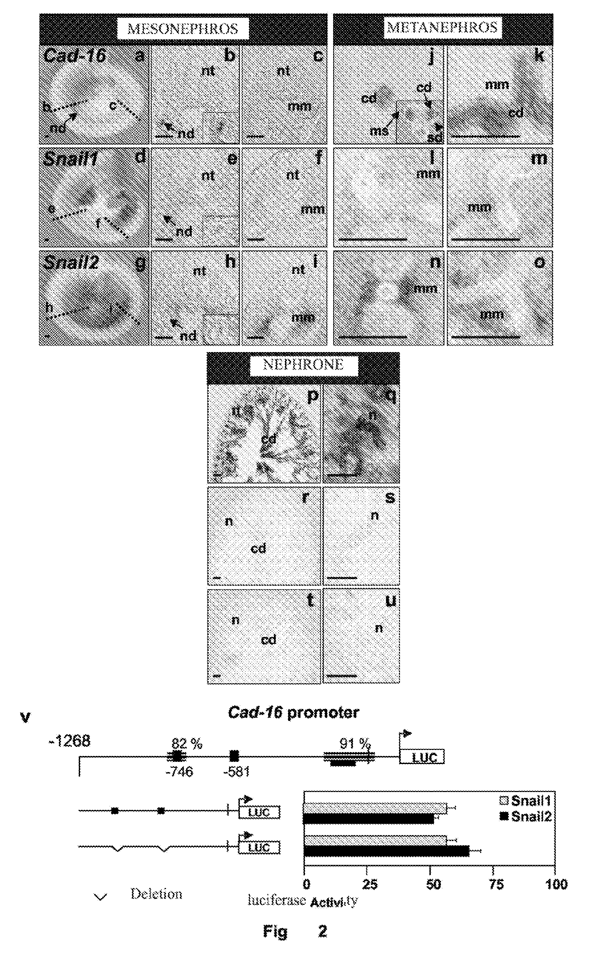

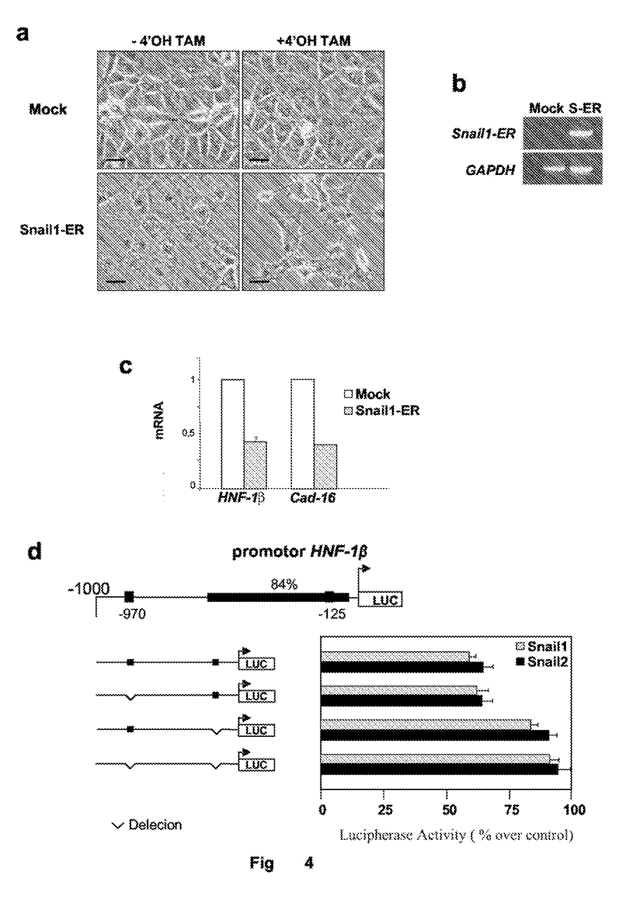

The data show that the expression patterns are complementary in the different embryo stages and that cadherin-16 only appears from the mesenchyme following the disappearance of expression of the Snail1 and Snail2 genes (Example 1). These data are consistent with the fact that Snail indirectly represses *in vivo* expression of the cadherin-16 gene (Figure 2v), through the repression of HNF-1beta and, more specifically, through direct action on the promoter thereof (binding to conserved consensus E-box identified in this invention), thereby inducing a complete EMT (Figure 4a and 4c).

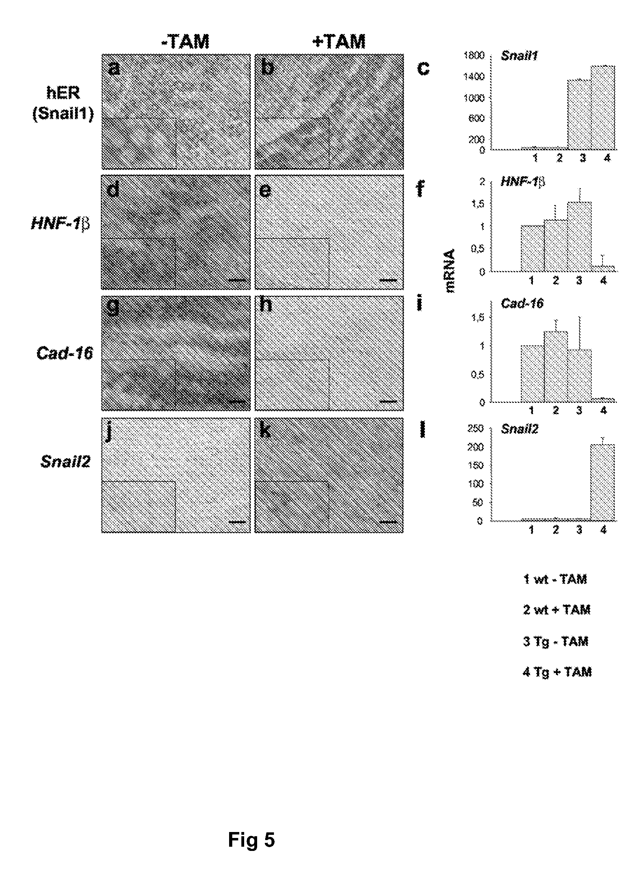

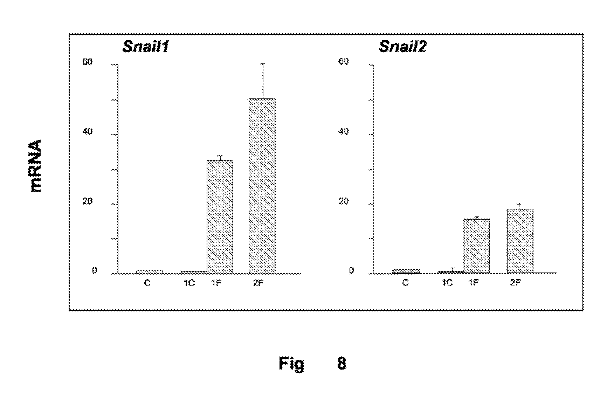

Moreover, in order to determine whether the Snail genes may repress HFN-1beta transcription and, consequently, Cadherin-16 expression, transgenic mice with inducible Snail1 activity were generated (Example 2). Thus, it was observed that Snail1 represses HNF-1beta *in vivo,* which induces repression of cadherin-16, and induces the loss of the cells' epithelial characteristics, which seem to acquire a morphology similar to the fibroblastic morphology that occurs in a complete EMT (Figure 5b, e, h, k, inserts). These changes disclosed herein are reminiscent of those observed following the experimental induction of renal fibrosis under different conditions (Liu, 2003). Finally, the results in patients with renal fibrosis showed that Snail expression causes the epithelium-to-mesenchyme transition (Example 3, Figure 8).

In summary, these data suggest that the Snail1 and 2 genes act as repressors of the epithelial phenotype in the mature kidney and, moreover, that the activation thereof is sufficient to induce all the characteristics of EMT and of renal fibrosis, i.e., that there is a direct relationship -not only a temporal association—between the activity of the Snail genes and the etiopathogeny of this disease. Thus, the presence of Snail may be considered to be a marker of renal fibrosis and, therefore, the identification thereof may be used as a diagnosis of renal fibrosis; and, on the other hand, the Snail protein may be of great utility in identifying new drugs for the treatment of renal fibrosis, and the gene inhibition thereof as a form of therapy. These therapeutic approaches to renal fibrosis are based on the use of compounds or agents that inhibit the activity of said Snail protein.

Therefore, an object of this invention is a method of identifying a renal fibrosis process, hereinafter renal fibrosis process identification method of the invention, based on the identification of the presence of Snail in a biological sample, which comprises the following steps:
a) identification of the presence of Snail, in a biological sample of renal origin, and
b) comparison of the presence of Snail observed in a) with the absence thereof in a control sample, and where its presence is indicative of the existence of renal fibrosis.

As used in this invention, the term "Snail genes" or "Snail proteins" refers to both the Snail1 gene or protein (SEQ ID NO 1 and 2, respectively) and the Snail2 gene or protein (SEQ ID NO 3 and 4, respectively), as well as any nucleotide or amino acid (aa) sequence that is analogous to those of other species, respectively. In the sense used in this description, the term "analogous" is intended to include any nucleotide or amino acid sequence that may be isolated or constructed on the basis of the nucleotide or aa. sequences shown in this specification, for example, by the introduction of conservative or non-conservative nucleotide or aa. substitutions, including the insertion of one or more nucleotides or aa., the addition of one or more nucleotides or aa. at any of the ends of the molecule or the deletion of one or more nucleotides or aa. at any end or in the interior of the sequence, and which is an encoding sequence or peptide with an activity similar to that of the sequences of the invention, i.e., that it is capable of inducing renal fibrosis.

In general, an analogous nucleotide or amino acid sequence is substantially homologous to the amino acid sequence previously discussed. In the sense used in this description, the expression "substantially homologous" means that the nucleotide or aa. sequences in question have a degree of identity of, at least, 40%, preferably of, at least, 85%, or more preferably of, at least, 95%.

A particular object of the invention is the identification method of the invention wherein the identification of Snail of a) relates to the human forms of Snail1 (hSnail1, SEQ ID NO 5 and 6) and (hSnail2, SEQ ID NO 7 and 8), whether the identification is in the form of a gene transcript (mRNA) or the protein form of both genes. These analyses designed to identify Snail expression levels may be performed by a person skilled in the field of biomedicine, thanks to the information disclosed in this invention and in the state of the art, by different techniques (Sambrook, J., Fritsch, E.F., and Maniatis, T. (1989). Molecular cloning: a laboratory manual, 2nd ed. Cold Spring Harbor Laboratory, Cold Spring Harbor, N.Y.).

Another particular object of the invention is the renal fibrosis process identification method wherein the identification of Snail is performed using specific Snail antibodies. The antibodies may be monoclonal or polyclonal.

Another particular object of the invention is the renal fibrosis process identification method wherein the identification of Snail is performed by *in situ* hybridisation with a Snail precursor.

Another particular object of the invention is the renal fibrosis process identification method wherein the identification of Snail is performed by RT-PCR of a Snail gene precursor. This method is based on the extraction of polyA+ RNA from a biological sample of renal origin and a control tissue and the amplification of the Snail-encoding sequence with suitable primer oligonucleotides.

On the other hand, this diagnostic method for renal fibrosis may be performed using Snail as the sole marker or jointly with other markers of renal fibrosis, for example as a part of a biological expression microarray, either in gene form -from mRNA— or in protein form, that defines a diagnostic marker or profile for pulmonary fibrosis.

Another object of this invention is a method of identifying and evaluating the activity of Snail protein inhibitory compounds which are useful to treat renal fibrosis, hereinafter compound identification method of this invention, which comprises the following steps:
a) Bringing into contact a biological system wherein there is a Snail expression that produces renal fibrosis with the candidate compound that is the object of this method, and incubation under suitable conditions,
b) determination of an indicative parameter of the renal fibrosis process, and
c) identification of a compound that inhibits Snail protein activity when a reduction of said renal fibrosis parameter is observed.

Another particular object of this invention is the identification method of the invention where the biological system of point a) is a transgenic animal where the expression of the Snail protein is inducible, in a constant or conditional manner, and where the expression thereof causes renal fibrosis. A particular embodiment is one wherein the transgenic animal is the transgenic mouse of this invention (Example 2, transgSnail1-ER mouse).

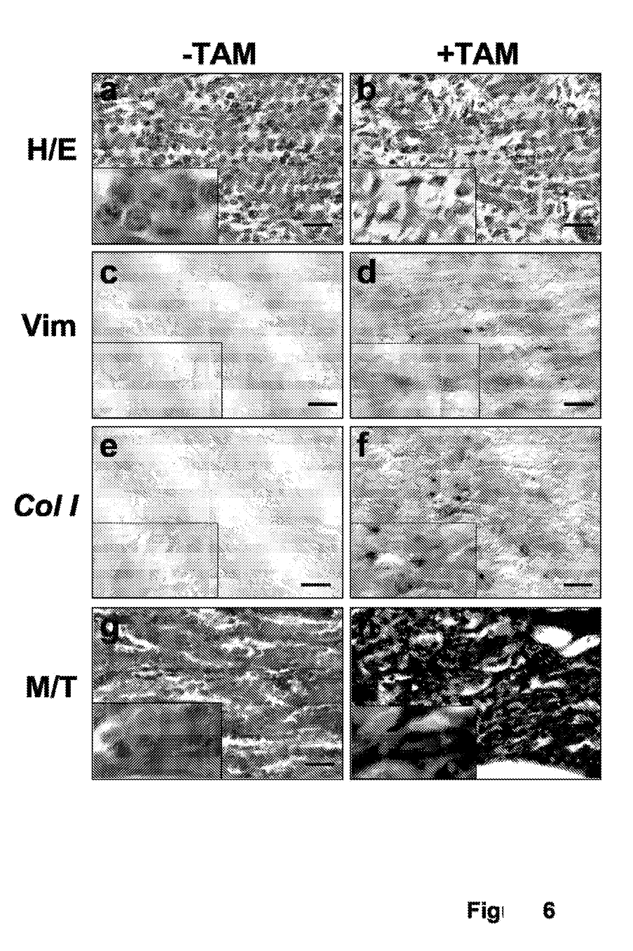

Another particular object of this invention is the identification method of the invention wherein the parameter related to the renal fibrosis process of a) belongs, for illustrative purposes, and without this limiting the scope of this invention, to the following group: a morphological change characteristic of an EMT, level of vimentin, Collagen I gene transcription and deposition of collagen fibres (Figure 6, Example 2).

Another object of this invention is the use of a compound or agent that inhibits Snail protein activity, hereinafter use of a compound of this invention, in the preparation of drugs or pharmaceutical compositions for the treatment of renal fibrosis, preferably human.

As used in this invention, the term "inhibitory or antagonist compound/agent" refers to a molecule which, when it is bound to or interacts with the Snail protein (for example, SEQ ID NO 2, SEQ ID NO 4, SEQ ID NO 6 and SEQ ID NO 8), or with functional fragments thereof, reduces or eliminates the intensity or the duration of the biological activity of said protein. This definition includes, furthermore, those compounds which prevent or reduce the expression of the Snail protein encoding gene, i.e., which prevent or reduce gene transcription, mRNA maturation, mRNA translation and post-translational modification. An inhibitory agent may be composed of a peptide, a protein, a nucleic acid or polynucleotide, a carbohydrate, an antibody, a chemical compound or any other type of molecule that reduces or eliminates the effect and/or the function of the Snail protein.

For illustrative purposes, said polynucleotide may be a polynucleotide that encodes a Snail protein gene or mRNA sequence specific anti-sense nucleotide sequence, or a polynucleotide that encodes a Snail protein mRNA specific ribozyme, or a polynucleotide that encodes a Snail protein mRNA specific aptamer, or a polynucleotide that encodes a Snail protein mRNA specific interference RNA ("small interference RNA" or siRNA).

The above-mentioned polynucleotides may be used in a gene therapy process which allows, by means of any technique or procedure, the integration thereof in the cells of a human patient. This objective may be achieved by administering a gene construct comprising one of the above-mentioned polynucleotides to these kidney cells in order to transform said cells, allowing for their expression in the interior thereof in such a way that Snail protein expression is inhibited. Advantageously, said gene construct may be included within a vector, such as, for example, an expression vector or a transfer vector.

As used in this invention, the term "vector" refers to systems used in the process of transferring an exogenous gene or an exogenous gene construct inside the cell, thereby allowing for the transport of exogenous genes and gene constructs. Said vectors may be non-viral or viral vectors (Pfeifer A, Verma IM (2001) Gene therapy: promises and problems. Annu Rev Genomics Hum Genet 2: 177-211) and the administration thereof may be prepared by a person skilled in the art on the basis of the needs and specificities of each case.

Therefore, in another particular embodiment of the invention, said use of a compound is based on the fact that the inhibitory compound is a nucleic acid or polynucleotide which prevents or reduces the expression of the human Snail protein encoding gene and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

Previously, antisense oligonucleotides have been disclosed and even patent protected (US20060003956, Materials and methods for the derepression of the E-cadherin promoter; Kajita M, McClinic KN, Wade PA. Aberrant expression of the transcription factors snail and slug alters the response to genotoxic stress. Mol Cell Biol. 2004, 24(17): 7559-66); also disclosed and patent protected are siRNAs that inhibit the expression thereof (Peinado H, Del Carmen Iglesias-de la Cruz M, Olmeda D, Csiszar K, Fong KS, Vega S, Nieto MA, Cano A, Portillo F. A molecular role for lysyl oxidase-like 2 enzyme in snail regulation and tumor progression. EMBO J. 2005, 24(19): 3446-58; Tripathi MK, Misra S, Chaudhuri G. Negative regulation of the expressions of cytokeratins 8 and 19 by SLUG repressor protein in human breast cells. Biochem Biophys Res Commun. 2005, 329(2): 508-15). On the other hand, these gene inhibition techniques, and, more specifically, transport of the compounds -antisense oligonucleotides, iRNA, ribozymes or aptamers—may be performed using nanoparticles, which increase the success rate of said transfer (Lu PV and Woodle MC, Adv Genet 54: 117-42, 2005; Hawker CJ and Wooley KL, Science 19 (309): 1200-5, 2005).

Thus, a particular embodiment of the invention is the use of an iRNA that preferably binds to the gatgcacatccgaagccac (SEQ ID NO 9) Snail mRNA fragment sequence or to another fragment which comprises the latter.

Nucleotide sequences a)-d) mentioned above prevent mRNA gene expression or mRNA expression in the Snail protein, and, therefore, destroy its biological function, and may be developed by a person skilled in the field of genetic engineering on the basis of the existing knowledge about transgenesis and gene expression destruction in the state of the art (Clarke, A.R. (2002) Transgenesis Techniques. Principles and Protocols, 2nd Ed. Humana Press, Cardiff University; Patent US20020128220. Gleave, Martin. TRPM-2 antisense therapy; Puerta-Fernández E et al. (2003) Ribozymes: recent advances in the development of RNA tools. FEMS Microbiology Reviews 27: 75-97; Kikuchi, et al., 2003. RNA aptamers targeted to domain II of Hepatitis C virus IRES that bind to its apical loop region. J. Biochem. 133, 263-270; Reynolds A. et al., 2004. Rational siRNA design for RNA interference. Nature Biotechnology 22 (3): 326-330).

On the other hand, the origin of these compounds that inhibit Snail protein activity may be varied, such that they may be of natural origin (for example, vegetable, bacterial, viral, animal origin, or from eukaryotic microorganisms) or synthetic.

Another object of this invention is a pharmaceutical composition or a drug for the treatment of renal fibrosis, hereinafter pharmaceutical composition of this invention, which comprises a therapeutically effective quantity of a compound or agent that inhibits the Snail protein, jointly with, optionally, one or more pharmaceutically acceptable adjuvants and/or vehicles.

A particular embodiment of this invention is a pharmaceutical composition wherein the inhibitory compound is a nucleic acid or polynucleotide which prevents or reduces the expression of the human Snail protein encoding gene and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

As previously discussed, a particular embodiment of the invention is the pharmaceutical composition of the invention wherein the Snail inhibitor is an iRNA that preferably binds to the gatgcacatccgaagccac (SEQ ID NO 5) Snail mRNA fragment sequence or to another fragment that comprises the latter.

The pharmaceutically acceptable adjuvants and vehicles that may be used in said compositions are the adjuvants and vehicles known by those skilled in the art and habitually used in the preparation of therapeutic compositions.

In the sense used in this description, the expression "therapeutically effective quantity" refers to the quantity of the agent or compound that inhibits Snail protein activity calculated to produce the desired effect and, in general, will be determined, amongst other factors, by the compounds' characteristics, including the patient's age, condition, the severity of the alteration or disorder, and the administration route and frequency.

In a particular embodiment, said therapeutic composition is prepared in solid form or in aqueous suspension, in a pharmaceutically acceptable diluent. The therapeutic composition provided by this invention may be administered by any suitable administration route; to this end, said composition will be formulated in the pharmaceutical form suitable for the chosen administration route. In a particular embodiment, the administration of the therapeutic composition provided by this invention is performed by parenteral route, by oral route, by intraperitoneal route, by subcutaneous route, etc. A review of the different pharmaceutical forms to administer drugs and the necessary excipients to obtain them may be found, for example, in "Tratado de Farmacia Galénica", C. Faulí i Trillo, 1993, Luzán 5, S.A. Ediciones, Madrid.

Another object of this invention is the use of the pharmaceutical composition of the invention in a treatment method for a mammal, preferably a human being, suffering from renal fibrosis, hereinafter use of the pharmaceutical composition of this invention, which consists of administering said therapeutic composition that inhibits the fibrosis process.

A particular object of this invention is the use of the pharmaceutical composition of this invention wherein the renal fibrosis is caused by a disease, disorder or pathology which, for illustrative purposes, and without this limiting the scope of the invention, belongs to the following group: IgA nephropathy, glomerulonephritis, diabetes, renal damage induced by toxicity, urinary obstruction and deterioration of kidney transplants.

### DESCRIPTION OF THE FIGURES

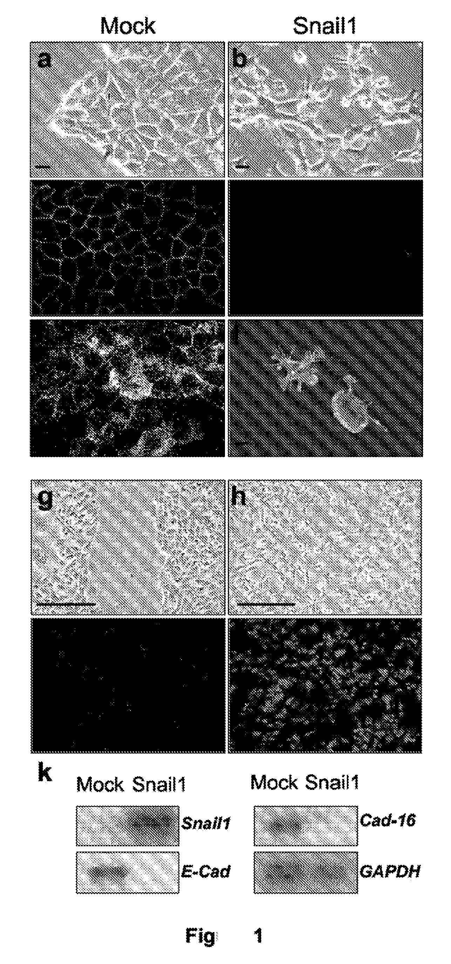

**Figure 1****. Snail induces EMT in NMuMG cells concomitant with Cadherin-16 repression.** Phase-contrast images (a, b), expression of E-Cadherin (c, d) and F-actin (e, f) in cells transfected with the empty vector (Mock) or transfected with Snail1 (Snail). The motility was determined by a cultured wound assay (g, h). The invasive properties were analysed by the cells' capacity to go through collagen IV gels. (k) Expression of E-Cadherin, cadherin-16 and Snail1 by RT-PCR in Mock and Snail cells. The cells that express Snail have lost E-Cadherin, reorganised the actin fibres and acquired a fibroblastic morphology, all of which is indicative of an EMT. Moreover, they are capable of closing the wound in 24 hours and of invading the collagen gels. Bar scale 50 µm.

**Figure 2****. The renal epithelia that express Cadherin-16 originate from positive mesenchyme for Snail genes.** *In situ* hybridisation for Cadherin-16, Snail1 and Snail2 on different days of the mouse's embryo development: 10.5 (a-i), 13.5 (j-or) and 17.5 (p-u). Cadherin-16 is expressed in the newly-formed nephric duct epithelium (nd, b), which no longer expresses Snail (e, h, inserts). Snail is observed in the undifferentiated metanephric mesenchyme (mm). The desiccated urogenital systems (see insert in j) or the sections thereof (j-or) were hybridised with probes in order to detect *Cadherin-16* and the *Snail* genes. *Cadherin-16* is also expressed in the mounds in formation (j, k), jointly with the sexual ducts (sd) and the transient mesonephros' epithelial mounds (ms), as shown in the insert in j. The Snail genes continue to be expressed in the remaining mesenchyme (mm, I-or). The nephrons' and the collecting tubules' epithelium expresses high Cadherin-16 levels when the entire mesenchyme has become differentiated into the epithelium and Snail gene expression has disappeared (r-u). This expression remains in the adult kidney. Bar scale 100 µm. v. Snail transcription factors inhibit Cadherin-16 gene promoter activity. Snail1 and Snail2 continue to repress promoter activity when the Snail binding sites have been eliminated.

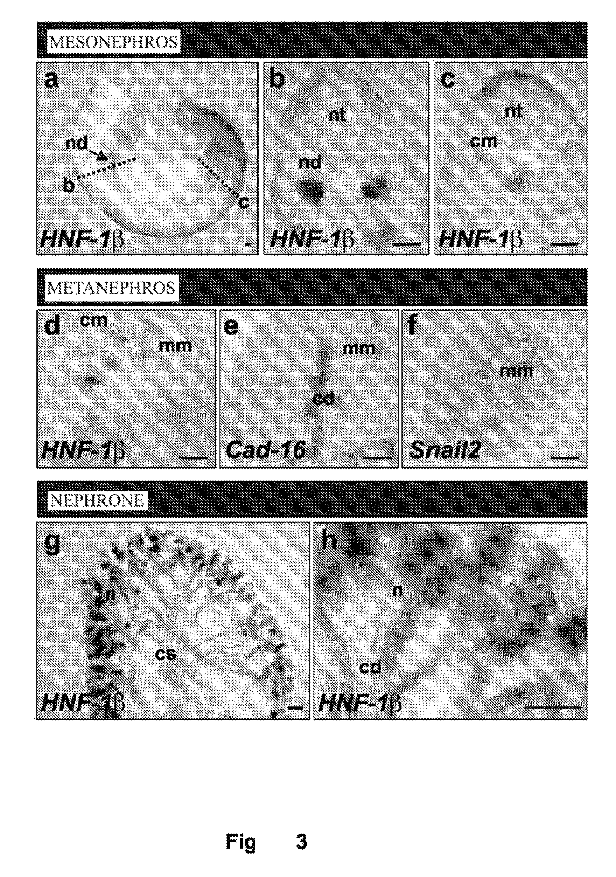

**Figure 3****.- The expression of HNF-1beta precedes that of Cadherin-16 in the developing kidney's epithelial components.** *In situ* hybridisation of embryos with 10.5, 13.5 and 17.5 days of development and their corresponding sections. *HNF-1beta* expression is observed as soon as mesenchyme-derived epithelia appear. At 10.5 days of development, the expression is observed in the nephric duct (b) and in the mesenchyme that is condensing in regions where Cadherin-16 expression has yet not appeared (Figure 2c). At 13.5 days of development, in addition to the nephric duct, many *HNF-1beta* expression sites are observed (d), some of which already express Cadherin-16. *Snail2* is expressed in the metanephric mesenchyme. Bar scale 100 µm

**Figure 4****.- Snail1 and Snail2 repress Cadherin-16 expression by repressing the transcription of the activator thereof, HNF-1beta. a)** The activation of Snail1 represses the expression of Cadherin-16 and HNF-1beta. The NMuMG cells stably transfected with an activatable version of Snail1 change their morphology 24 hours after the induction. **b)** Transgene expression by RT-PCR. c) Real-time RT-PCR of *Cadherin-16* and *HNF-1beta* 24 hours after administration of the inducer, 4-OH-tamoxifen. And d) Diagram of the 1-kb region in front of the mouse HNF-1beta gene translation initiation site, showing a very conserved region with humans. Both Snail binding sites are indicated by black boxes. Snail1 and Snail2 repress the HNF-1beta native promoter activity, but did not affect the activity of a promoter whose conserved site was eliminated.

**Figure 5****.- Snail1 represses *in vivo* expression of *HNF-1beta* and cadherin-16. a, b)** The exogenous Snail protein was translocated to the nucleus following the administration of tamoxifen, as observed by the anti-human-estrogen receptor antibody. c) Real-time RT-PCR of Snail1 in normal and transgenic mice in the absence or presence of tamoxifen. Expression of *HNF-1beta* (d, e), cadherin-16 (g, h) and *Snail2* (j, k) in marrow sections of two-week-old transgenic kidneys and the corresponding expression values by real-time RT-PCR (f, i and I, respectively). Bar scale 25 µm. Although it is not shown, E-Cadherin, a direct target of Snail shown in tissues with a different etiology, also disappears (reviewed in Barrallo-Gimeno and Nieto, 2005).

**Figure 6****.- Snail activation is sufficient to induce renal fibrosis in transgenic mice.** Snail activation induces a morphological change characteristic of an EMT (a, b); activation of the mesenchymal marker vimentin (c, d); activation of Collagen I gene transcription (e, f) and deposition of collagen fibres, as observed by means of the Trichrome-Masson stain (g, h).

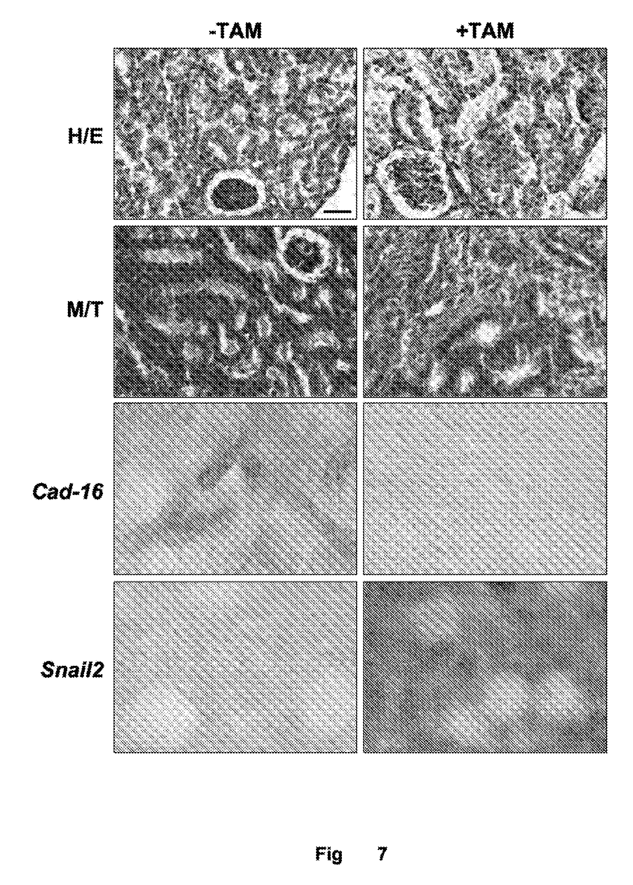

**Figure 7****.- Snail activation also produces fibrosis in the renal cortex.** The same morphological changes indicative of EMT are observed as in the marrow; also observed is the disappearance of Cadherin-16 and E-Cadherin, and the appearance of Snail2 and of Collagen 1 deposits.

**Figure 8****.- Human kidneys with fibrosis show strong Snail expression.** Tissues from normal human kidneys and from patients with pulmonary fibrosis subject to nephrectomy for renal tumour exeresis and for urinary obstruction and renal failure, respectively, were analysed. Shown is an RT-PCR quantitative analysis of Snail1 and Snail2 expression in normal human kidney tissue (C=control, n=4), non-fibrotic tissue from a kidney with fibrosis (1C) and fibrotic tissue from patient 1 (1F) and patient 2 (2F). The transcription levels were normalised with the GAPDH mRNA expression levels and the error bars represent the standard error from the mean.

### EXAMPLES OF THE INVENTION

### Example 1.- Snail genes repress cadherin-16 expression by repressing the gene transcription of the activator thereof, HNF1-beta.

The ectopic expression of Snail1 in the NMuMG cell line induced an EMT, as has been previously described in other epithelial cell lines (Cano et al., 2000; Batlle et al., 2000), with the acquisition of mesenchymal characteristics, such as the loss of E-cadherin expression (Figure 1d), the reorganisation of the F-actin cytoskeleton (Figure 1f); moreover, they acquire mobility (Figure 1g and 1h) and invasive properties (Figure 1i and 1j). In addition to repressing known epithelial markers, it also repressed the expression of Cadherin-16 (Figure 1k), a kidney-specific cadherin. Since the mouse breast and other cell lines from human breast did not express Cadherin-16, it was concluded that this expression is peculiar to the NMuMG cell line, but it induced us to study whether Snail can repress Cadherin-16 in the kidney.

In order to determine whether both Snail genes can repress Cadherin-16 *in vivo,* the relative expression patterns were studied during embryo development in mice, when different EMT and MET processes take place which lead to the formation of the mature kidney. As soon as the nephric ducts epithelialise, Snail2 expression is repressed and cadherin-16 clearly appears; it can be observed that the antero-posterior gradient differentiation in the ducts correlates with the complementary expression of the cadherin-16 and Senil genes (Figure 2a-2i). The collecting ducts induce the nephric mesenchyme to condense and differentiate in tubular structures which will give rise to the nephrons. The nephric mesenchyme expresses both genes, Snail1 and Snail2 (Figure 2f and 2i), and, again, the epithelialisation thereof correlates with their repression and with the beginning of cadherin-16 expression (Figure 2j-2o). On day 17.5 dpc, in addition to the facts mentioned regarding the collecting ducts, strong cadherin-16 expression can be observed in the neuron (Figure 2p-2u). At this time, the Snail genes are completely repressed in the kidney, without an undifferentiated mesenchyme being observed, a situation which remains throughout adult life. The data show that the expression patterns are complementary in the different embryo stages and that Cadherin-16 only appears from mesenchyme following the disappearance of the expression of the Snail1 and Snail2 genes. These data are consistent with the fact that Snail represses Cadherin-16 gene expression *in vivo.*

In order to determine whether Snail directly repressed the cadherin-16 gene transcription, its promoter was analysed; there, two Snail transcription factor-binding consensus E-boxes, located on-site in positions -581 and -746 (Figure 2v), were found. When Snail1 or Snail2 were transfected jointly with a gene construct containing these two boxes, and capable of reproducing the Cadherin-16 expression pattern (Whyte DA et al., 1999; Shao X et al., 2002), a 61% and 56% reduction was observed, respectively, in the promoter activity. In order to confirm whether the Snail binding boxes were necessary to produce this effect, constructs which had these boxes mutated or eliminated were co-transfected with Snail1 or Snail2. These experiments produced the same result, which indicates that Snail represses the Cadherin-16 promoter activity indirectly (Figure 2v).

Since the Snail genes are characterised as repressors, we studied whether their repressor effect took place through the repression of a Cadherin-16 activator. HNF-1beta is a known potent Cadherin-16 activator (Bai et al., 2002) and, furthermore, it exhibits a very interesting expression pattern during kidney development. Thus, we studied its expression pattern in relation to that of the Snail genes and Cadherin-16, and an expression complementary to that of the Snail genes was observed, which appears when the Snail genes disappear; it was also observed that the expression thereof occurs prior to that of Cadherin-16 and in the same territories. Thus, at 10 dpc, HNF-1beta is expressed in the differentiation of the nephric ducts in the posterior region (F igura 3c), where the Snail genes have been repressed and cadherin-16 is not expressed (Figure 2c). At 13.5 dpc, the metanephric mesenchyme expresses Snail1 and Snail2, whereas the HNF-1beta transcripts are detected in some areas of epithelialised mesenchyme which are still negative for cadherin-16 (Figure 3d-f). The nephrons that differentiate from that mesenchyme to give rise to the functional epithelial structures of the mature kidney express both HNF-1beta and cadherin (Figure 2). These data are consistent with the fact that Snail represses HNF -1beta and that this repression prevents the appearance of cadherin-16.

In order to determine whether the acute activation of Snail is capable of repressing HNF-1beta and cadherin-16, an inducible construct that activates Snail1 by the administration of 4-OH-Tamoxifen was used (similar to that used in Locascio et al., 2002) in NMuMG cells. This method is used for the quick activation of transcription factors, since the protein is synthesised in inactive form and the inducing agent simply transports it to the cell nucleus so that it may act as a transcription factor, regulating the expression of other genes. These experiments made it possible to state that the activation of the Snail1 protein was sufficient to repress HNF-1beta and cadherin-16 transcription in 24 h, thereby inducing a complete EMT (Figure 4a and 4c). Interestingly, 6 hours after Snail activation, a 20% repression of HNF-1beta levels was observed, together with unaltered cadherin-16 values. These data indicate that Snail induces the sequential repression of HNF-1beta and cadherin-16, i.e., that Snail inhibits cadherin-16 expression through the repression of HNF-1beta.

In order to determine whether HNF-1beta repression by Snail takes place directly on the promoter, similar experiments to those described on the Cadherin-16 promoter were performed with Snail1 and Snail2. Two Snail-binding consensus E-boxes were identified, one of them conserved in the mouse and human promoter (Figure 4d). The co-transfection experiments with the construct of this intact HNF-1beta promoter showed that both genes, Snail1 and 2 -as previously described for the cadherin-16 promoter—, repress the promoter activity (Figure 4d), but this is not the case with those wherein the two boxes, or simply the conserved box, had been eliminated (Figure 5d). These data show that Snail is a direct repressor of HNF-1beta gene transcription by binding with the conserved box.

### Example 2.- Snail induces renal fibrosis in transgenic mice

In order to determine whether the Snail genes can repress HNF-1beta transcription and, consequently, the expression of Cadherin-16 and also of E-Cadherin (its known target in other tissues (reviewed in Barrallo-Gimeno and Nieto, 2005) in the kidney, transgenic mice with inducible Snail1 activity were generated (transgSnail1-ER mouse). The same system as for inducible expression in cell lines was used. Normal and transgenic mice were treated, after birth, with an injection of excipient or with tamoxifen for two weeks, when they were sacrificed and the expression (Figure 5c) and the location of the transgenic protein (Figure 5a and 5b) were analysed. The analysis of the corresponding kidneys showed the translocation of the exogenous protein to the nucleus (Figure 5b) and the repression of E-Cadherin, Cadherin-16 and HNF-1beta gene expression only in the transgenic mice treated with tamoxifen (Figure 5d-5i; supplementary figure 5 online). Also observed was induction of the expression of Snail2, the member of the family that has prominent expression during kidney development (Figure 5j-5l; supplementary figure 5 online) and has been shown to be functionally equivalent to Snail1 in cell lines and in embryos as an EMT inducer (Bolós et al., 2003; del Barrio and Nieto, 2002). In summary, it was observed that Snail1 represses HNF-1beta *in vivo,* which induces the repression of cadherin-16. Jointly with this repression of E-cadherin, Snail1 expression has a great impact on the loss of epithelial characteristics, such that the marrow collecting duct cells seem to acquire a fibroblastic-like morphology reminiscent of EMT (Figure 5b, e, h, k, inserts).

In order to verify that the expression of the Snail genes not only induced the repression of the above-mentioned genes, but a complete EMT, the histological phenotype of these transgenic animals' kidneys was analysed, and it was observed that the activation of Snail1 induced a morphological change in the kidney marrow consistent with EMT, activation of the mesenchymal marker vimentin and activation of Collagen I transcription (Figure 6e-f), jointly with the deposits thereof (Figure 6g-h), a prototypical marker of renal fibrosis (Alexakis et al., 2005). These changes also appeared in the renal cortex (Figure 7). The changes described herein are reminiscent of those observed following the experimental induction of renal fibrosis under different conditions (Liu, 2003). These data indicate that the Snail1 and Snail2 genes act as repressors of the epithelial phenotype in the kidney and, moreover, that the activation thereof is sufficient to induce all the characteristics of renal fibrosis.

### Example 3.- The Snail genes pathologically activate during the human renal fibrosis process.

Samples of normal and fibrotic renal tissue from human kidneys obtained from patients subject to nephrectomy, more specifically, of renal tissue obtained from non-tumour renal areas in patients subject to nephrectomy (4 cases), and of fibrotic and non-fibrotic areas in patients suffering from renal failure caused by urinary obstruction, were analysed. The samples, of approximately 1 cm3, were fixated in 10% formalin or immediately frozen with isopentane in liquid nitrogen. The Research Ethics Committee of the Sant Joan Hospital of Alicante, where the samples were obtained, approved the protocols to analyse the tissues, which were subject to real-time RT-PCR analysis.

The quantitative RT-PCR was performed using the ABI PRISM® 7000 detection system and TaqMan® probes. The oligos and the probes were obtained from Applied Biosystems Assays-on-Demand, from the catalogue: Hs 99999905-m1 (GAPDH), Hs 00193591-m1 (SNAl1), Hs 00161904-m1 (SNAI2), Hs 00187880-m1 (CADH16). The RNA expression was calculated using the comparative Cₜ method normalised with the GAPDH levels. The data are expressed in relation to a calibrator (mock or wild mouse cells) using the formula 2^{-(ΔΔCt)}±SD. The RNAs were extracted and the cDNA was synthesised from the human kidney samples.

The histological characteristics of renal fibrosis were observed in the samples from patients suffering from renal failure (data not shown). On the contrary, no Snail1 or Snail2 transcript expression was observed in the normal human tissue (Figure 7; n=4), whereas high Snail1 levels were detected in patients with renal fibrosis (Figure 7).

As in the case of the animal models, Snail2 was expressed in the fibrotic tissue. Interestingly, the presence of fibrotic and non-fibrotic tissue in the same kidney shows that Snail activation is only associated with fibrosis (Figure 7). In conclusion, it may be affirmed that human renal fibrosis is accompanied by the aberrant activation of Snail expression, leading to significant effects in the kidney's epithelial homeostasis. Snail1 activation in the adult kidney was sufficient to cause the epithelium-to-mesenchyme transition of the tubular cells and the collecting ducts, and to favour the deposit of collagen; therefore, Snail may be considered to be a good candidate for a therapeutic target or prognostic and diagnostic marker of fibrosis and the cyst formation that takes place in human renal diseases.

### MATERIALS AND METHODS

**Plasmids and antibodies.** *Expression plasmids.* pcDNA3-Snail1 corresponds to the complete mouse *Snail1* cDNA sequence inserted into plasmid pcDNA3 (Invitrogen; Cano et al., 2000). pcDNA3-Snail-ER corresponds to the Snail1-encoding sequence joined to a mutated version of the binding domain to the human estrogen receptor agonist that recognises the 4'-OH-Tamoxifen synthetic ligand (Locascio et al., 2002). The complete mouse *Snail2* cDNA sequence was also cloned in pcDNA3 (pcDNA3-*Snail2*). *Reporter plasmids.* The pKsp(1268F)-Luc reporter construct of the mouse *cadherin-16* promoter was gently supplied by Doctor Peter Igarashi (University of Texas, Southwestern Medical Center, Dallas, TX). The mouse *HNF-1*β promoter sequence, containing 1,072 pb from the ATG, was amplified by PCR from the genomic DNA of NMuMG cells using high-fidelity DNA polymerase (*PfuTurbo,* Stratagene). The primer oligonucleotides used for the amplification were 5'-ggtaccATCTACACATTCACTACTAGA-3' (SEQ ID NO 10) and 5'-acgcgtTTTCCAAGGACGGAAAAAGAA-3' (SEQ ID NO 11), corresponding to the GenBank^{™} X55842 sequence and containing the *Kpnl* and *Mlul* restriction sites at the 5' ends, respectively. The purified PCR product was subcloned in vector pGL3-basic (Promega). The Quickchange Site Directed Mutagenesis kit (Stratagene) was used to introduce mutations inside the E-boxes present in the mouse *cadherin-16* promoter. The 5'-CA(G/C)(G/C)TG-3' sequence was mutated by 5'-AA(G/C)(G/C)TA-3'. The sequences of the oligonucleotides used to eliminate the E-boxes in the *cadherin-16* and *HNF-1β* promoters are available upon request. *Antibodies:* Anti-E-cadherin (ECCD2, 1:200, Takara), anti-vimentin (M0725, 1:200, Dako). The Snaill-ER fusion protein was detected by immunoblots or immunohistochemistry using an anti-α human estrogen receptor antibody (1:100, Santa-Cruz). F-actin was detected using phalloidin-FITC (1:10, Sigma).

**Cell culture and generation of stably transfected cells with *Snail1* or *Snail1-ER.*** The NMuMG cell line comes from the epithelium of mouse mammary gland. This epithelial cell line expresses high E-cadherin levels and is very sensitive to the epithelium- mesenchyme transition induced by TGFβ (Miettinen et al., 1994). For the transfection, the NMuMG cells were seeded in 6-well plates (5x10⁴ cells per 3.5-cm well) in a 1:1 mixture of Ham's F12 medium and Dulbecco's Modified Eagle's medium supplemented with 100 IU/ml of penicillin, 0.1 mg/ml of streptomycin, 2 mM of glutamine and 2.5 µg/ml of amphothericin B, 10% of fetal bovine serum and 10 µg/ml of insulin. 24 hours after seeding the cells, 500 ng of DNA were added in the presence of LIPOFECTAMINE (Roche) and the cells were incubated with the DNA overnight. One day later, selection of the neomycin-resistant transfected cells began, using neomycin analogue G-418 (Calbiochem, 400 µg/ml). Two independent clones transfected with *Snail1* or *Snail1-ER* were isolated.

**Migration and invasion assays.** The cells were seeded in 6-well plates at a density of 3x10⁵ cells per well. After 24 hours, a wound was made in the central area of the confluent culture and the cells were incubated for an additional 24-hour period after washing the culture and adding fresh medium thereto. The cultures were observed at several times and photographs were taken of the wounded area using a Zeiss Axiovert inverted microscope. The invasion assays in type IV collagen gels were performed in Boyden chambers, as described in Cano et al., 2000. The cells in the lower compartment were collected and counted after 24 hours. Parallel to this, the nuclei of the cells on the lower part of the filter were stained with DAPI following fixation in methanol and after having removed all the cells from the upper part of the filter.

**Microarray genetic analysis.** The Affymetrix murine genome Chip U74Av2 was used to define the gene expression profile of the cells transfected with *Snail1* (*Snail1*-transfectants). This profile was compared to that of the cells transfected with the empty vector (mock-transfectants). The biotinylated RNAs were analysed and hybridised to the Chip. Subsequently, the Chip was stained with streptavidin-phycoerithrin and scanned. The chips were analysed using the Affymetrix® Microarray Suite 5.0 programme.

**Transcript analysis.** The Poly(A)+ mRNAs were extracted from the NMuMG cells using the Microfast Track kit (Invitrogen). For the Northern blot analysis, 1.5-µg aliquots of Poly(A)+ mRNAs purified with oligo(dT) cellulose were transferred to nylon membranes that were hybridised with [α-³²P]dCTP-radiolabelled probes (rediprime II, Amersham Biosciences). The Snail1, E-cadherin, cadherin-16 and GAPDH DNA probes were amplified by RT-PCR from 25 ng of the corresponding purified cDNAs and the hybridisations thereof were visualised by autoradiography using a Hyperfilm MP (Amershan Biosciences). The RT-PCR for Snail1 and GAPDH was described in Cano et al., 2000. The real-time RT-PCR was performed using the ABI PRISM® 7000 sequence detection system and TaqMan® probes. The oligonucleotides and the probes were obtained from Applied Biosystems Assays-on-Demand, as follows: Mm-99999915-g1 (GAPDH), Mm-00441533-g1 (Snail1), Mm00483196-m1 (Cadherin-16) and Mm-00447452-m1 (HNF-1β). The RNA expression was calculated using the comparative Cₜ method normalised with GAPDH. The final results are expressed in relation to a calibrator (mock or wild mouse cells) using the formula 2^{-(ΔΔCt)}±SD. The RNA was extracted and the cDNA was synthesised from the cells transfected with *Snail1-ER* or with the empty vector at several times following treatment with 4'-OH-Tamoxifen and from the kidneys of the newborn normal or transgenic animals or two weeks after the administration of tamoxifen or of an excipient.

***In situ* hybridisation.** The mouse embryos came from the Balb-C strain, and their ages, established in days post-coitum (dpc) were determined considering the day when the vaginal plug is seen as day 0.5. The urogenital systems or the kidneys were desiccated at 13.5 dpc and 17.5 dpc, respectively, and fixated in 4% paraformaldehyde in PBS/DEPC overnight. Subsequently, they were processed directly for *in situ* hybridisation (ISH) or soaked in gelatin and cut with a vibratome in order to obtain 50-µm sections. The ISHs in gelatin sections or in intact embryos were performed as described in Blanco et al., 2002 using the DIG-11-UTP-labelled mouse Snail1, Snail2 and E-cadherin RNA probes (Cano et al., 2000; Sefton et al., 1998). The probes for mouse Cadherin-16 and HNF-1β were obtained by RT-PCR from the cDNA of the NMuMG cells using the oligonucleotides described above. Following the hybridisation, the embryos or the kidney sections were processed as described in Cano et al., 2000.

**Promoter analysis.** The activity of the *cadherin-16* and *HNF-1*β promoters was determined by co-transfecting the NMuMG cells with 50 ng of pcDNA3-Snail, pcDNA3-mSnail2 or the empty vector, and with 300 ng of pKsp(1268F)-Luc or 400 ng of pmHNF-1β-*Luc*. A plasmid with the *Renilla reniformis luciferase* gene (phRL -CMV-Luc, Promega) was co-transfected as an efficiency control. 24 hours after the transfection, the activity of the firefly (Luc) and *renilla* luciferases was determined using the Dual Luciferase Reporter Assay system (Promega), following the supplier's instructions. The Luc activity was normalised to that of the *renilla* luciferase. In all the experiments, the total quantity of transfected DNA was standardised by adding the empty vector. The results are represented as the percentage of luciferase activity relative to the controls (luciferase values in cells co-transfected with the empty vector).

**Transgenic mice.** The Snail1-ER transgene was designed as previously described (Locascio et al., 2002) and a transgenic mouse (transgSnail1-ER mouse) was generated for this construct following standard procedures (Hogan, B., Beddington, R. and Lacy, F. Manipulating the mouse embryo. A laboratory manual. Cold Spring Harbor Laboratory Press (1994)). For this study, we selected an animal line that had a very high expression of the transgenic protein in the kidney. In this model, even though the Snail1-ER protein is constitutively expressed, its function as a transcription factor develops only when the protein is translocated in the nucleus following treatment with tamoxifen. The transgene is detected from the animal tails' DNA by PCR (the details about the oligonucleotides used are available upon request). The protein's subcellular location was analysed by immunohistochemistry using an anti-human estrogen receptor antibody. The same antibody was used to assess the quantity of Snail1-ER protein in the different tissues from the transgenic mice by Western Blots. The tamoxifen (Sigma) was first dissolved in ethanol (10% of the final volume) and, subsequently, in corn oil (Sigma) in order to obtain a final concentration of 30 mg/ml. The solution was sonicated in order to improve its solubility and 3 mg of Tamoxifen for every 20 grams of body weight were administered subcutaneously to the newborn animals every three days for two weeks. Once the treatment was concluded, the animals were sacrificed and the kidneys obtained were soaked in gelatin, cut with a vibratome for the ISH or immunohistochemistry or processed for the extraction of RNAs.

### References

- Bai, Y., Pontoglio, M., Hiesberger, T., Sinclair, A.M. & Igarashi, P. Regulation of kidney-specific Kspcadherin gene promoter by hepatocyte nuclear factor-1 beta. Am. J. Physiol. Renal Physiol. 283, F839-51 (2002).
- Barrallo-Gimeno, A. and Nieto, M.A. The Snail genes as inducers of cell movement and survival: implications in development and cancer. Development 132, 3151-61 (2005).
- Batlle, E. et al. The transcription factor snail is a repressor of E-cadherin gene expression in epithelial tumour cells. Nat. Cell Biol. 2, 84-9 (2000).
- Bolos, V. et al. The transcription factor Slug represses E-cadherin expression and induces epithelial to mesenchymal transitions: a comparison with Snail and E47 repressors. J. Cell Sci. 116, 499-511 (2003).
- Cano, A. et al. The transcription factor snail controls mesenchymal-epithelial transitions by repressing Ecadherin expression. Nat. Cell Biol. 2, 76-83 (2000).
- Chilosi et al., 2003.
- del Barrio, M.G. and Nieto, M.A. Overexpression of Snail family members highlights their ability to promote chick neural crest formation. Development 129, 1583-93 (2002).
- Dressler, 2002. Book.
- Hawker, C.J. and Wooley, K.L. The convergence of synthetic organic and polymer chemistries. Science 19 (309): 1200-5 (2005).
- Huber et al. Current Op Cell Biol (2005).
- Iwano, M. et al. Evidence that fibroblasts derive from epithelium during tissue fibrosis. J. Clin. Invest. 110, 341-50 (2002).
- Jinde et al.
- Kalluri and Neilson, 2003.
- Li et al., 2005.
- Locascio, A., Vega, S., de Frutos, C.A., Manzanares, M. and Nieto, M.A. Biological potential of a functional human SNAIL retrogene. J. Biol. Chem. 277, 38803-9 (2002).
- Rastaldi et al., 2002.
- Lu, P.V. and Woodle, M.C. In vivo application of RNA interference: from functional genomics to therapeutics. Adv Genet 54: 117-42 (2005).
- Sato, M., Muragaki, Y., Saika, S., Roberts, A.B. and Ooshima, A. Targeted disruption of TGF-beta1/Smad3 signaling protects against renal tubulointerstitial fibrosis induced by unilateral ureteral obstruction. J. Clin. Invest. 112, 1486-94 (2003).
- Shao, X., Johnson, J.E., Richardson, J.A., Hiesberger, T. and Igarashi, P. A minimal Ksp-cadherin promoter linked to a green fluorescent protein reporter gene exhibits tissue-specific expression in the developing kidney and genitourinary tract. J. Am. Soc. Nephrol. 13, 1824-36 (2002).
- Thompson, R.B. et al. Isolation and cDNA cloning of Kspcadherin, a novel kidney-specific member of the cadherin multigene family. J. Biol. Chem. 270, 17594-601 (1995).
- Vongwiwatana et al., 2005.
- Whyte, D.A. et al. Ksp-cadherin gene promoter. I. Characterization and renal epithelial cell-specific activity. Am. J. Physiol. 277, F587-98 (1999).
- Yáñez-Mo et al., 2002.
- Zeisberg, M. and Kalluri, R. The role of epithelial-to-mesenchymal transition in renal fibrosis. J. Mol. Med. 82, 175-181 (2004).

## Claims

1. A method of identifying a renal fibrosis process **characterised in that** it is based on the identification of the presence of Snail in a biological sample and that it comprises the following steps:
a) identification of the presence of Snail, in a biological sample of renal origin, and
b) comparison of the presence of Snail observed in a) with the absence thereof in a control sample, and where its presence is indicative of the existence of renal fibrosis.

2. A method according to claim 1, **characterised in that** the Snail of a) relates to the identification of a human Snail1 gene (SEQ ID NO 5) or Snail2 gene (SEQ ID NO 7) transcript.

3. A method according to claim 1, **characterised in that** the Snail of a) relates to the identification of the human Snail1 protein (SEQ ID NO 6) or the human Snail2 protein (SEQ ID NO 8).

4. A method according to claim 1, **characterised in that** the identification of the Snail protein of a) is performed using specific, monoclonal or polyclonal, Snail antibodies.

5. A method according to claim 1, **characterised in that** the identification of the Snail protein of a) is performed by *in situ* hybridisation with a Snail precursor.

6. A method according to claim 1, **characterised in that** the identification of the Snail protein of a) is performed by RT-PCR amplification of a Snail gene precursor.

7. Method of identifying and evaluating the activity of Snail protein inhibitory compounds that are useful for the treatment of renal fibrosis, **characterised in that** it comprises the following steps:
a) Bringing into contact a biological system wherein there is a Snail expression that produces renal fibrosis with the candidate compound that is the object of this method, and incubation under suitable conditions,
b) determination of an indicative parameter of the renal fibrosis process, and
c) identification of a compound that inhibits Snail protein activity when a reduction of said renal fibrosis parameter is observed.

8. Identification method according to claim 7, **characterised in that** the biological system of point a) is a transgenic animal where the expression of the Snail protein is inducible, in a constant or conditional manner, and where the expression thereof causes renal fibrosis.

9. Identification method according to claim 8, **characterised in that** the transgenic animal is the transgSnail1-ER mouse.

10. Identification method according to claim 7, **characterised in that** the parameter related to the renal fibrosis process of b) belongs to the following group: a morphological change characteristic of an EMT, level of vimentin, Collagen I gene transcription and deposition of collagen fibres.

11. Use of a compound or agent that inhibits Snail protein activity in the preparation of drugs or pharmaceutical compositions for the treatment of renal fibrosis, preferably human.

12. Use of a compound according to claim 11, **characterised in that** the compound is a nucleic acid or polynucleotide which prevents or reduces the expression of the human Snail protein encoding gene, either Snail1 or Snail2, and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

13. Use of a compound according to claim 12, **characterised in that** the iRNA is preferably bound to the Snail mRNA fragment sequence (SEQ ID NO 9) or to another fragment that comprises the latter.

14. Pharmaceutical composition or a drug for the treatment of renal fibrosis **characterised in that** it comprises a therapeutically effective quantity of a compound or agent that inhibits the Snail protein, either Snail1 or Snail2, jointly with, optionally, one or more pharmaceutically acceptable adjuvants and/or vehicles.

15. Pharmaceutical composition according to claim 14, **characterised in that** the inhibitory compound is a nucleic acid or polynucleotide which prevents or reduces the expression of the human Snail protein encoding gene, either Snail1 or Snail2, and includes a nucleotide sequence selected from:
a) a Snail protein gene or mRNA sequence specific anti-sense nucleotide sequence,
b) a Snail protein mRNA specific ribozyme,
c) a Snail protein mRNA specific aptamer, and
d) a Snail protein mRNA specific interference RNA (iRNA).

16. Pharmaceutical composition according to claim 15, **characterised in that** the iRNA is preferably bound to the Snail mRNA fragment sequence (SEQ ID NO 9) or to another fragment that comprises the latter.

17. Use of the pharmaceutical composition according to claims 14 to 16, in a treatment method for a mammal, preferably a human being, suffering from renal fibrosis, which consists of administering said therapeutic composition that inhibits the fibrosis process.

18. Use of the pharmaceutical composition according to claim 17, **characterised in that** the renal fibrosis is caused by a disease, disorder or pathology that belongs to the following group: glomerulonephritis, IgA nephropathy, diabetes, renal damage induced by toxicity, urinary obstruction and deterioration of kidney transplants.


Sequence Listing

<110>  CONSEJO SUPERIOR DE INVESTIGACIONES CIENTÍFICAS
       UNIVERSIDAD MIGUEL HERNANDEZ DE ELCHE

<120>  A METHOD FOR IDENTIFYING A RENAL FIBROSIS PROCESS,
       USE OF SNAIL-ACTIVITY-INHIBITING COMPOUNDS IN THE PRODUCTION
       OF PHARMACEUTICAL COMPOSITIONS, METHOD OF IDENTIFYING
       SAID INHIBITING COMPOUNDS, SAID PHARMACEUTICAL
       COMPOSITIONS AND APPLICATIONS THEREOF


<130>  Snail

<160>  11

<170>  PatentIn version 3.3

<210>  1
<211>  1613
<212>  DNA
<213>  Mouse


<220>
<221>  CDS
<222>  (64)..(858)
<223>  Mouse Snail1 encoding sequence

<400>  1
cggagttgac taccgacctt gcgcgacccg gtgaccccga ctacctaggt cgctctggcc      60

aac atg ccg cgc tcc ttc ctg gtc agg aag ccg tcc gac ccc cgc cgg      108
    Met Pro Arg Ser Phe Leu Val Arg Lys Pro Ser Asp Pro Arg Arg
    1               5                   10                  15

aag ccc aac tat agc gag ctg cag gac gcg tgt gtg gag ttc acc ttc      156
Lys Pro Asn Tyr Ser Glu Leu Gln Asp Ala Cys Val Glu Phe Thr Phe
                20                  25                  30

cag cag ccc tac gac cag gcc cac ctg ctg gcc gcc atc cct ccg ccc      204
Gln Gln Pro Tyr Asp Gln Ala His Leu Leu Ala Ala Ile Pro Pro Pro
            35                  40                  45

gag gtc ctc aac ccc gcc gct tcg ctg ccc acc ctc atc tgg gac tct      252
Glu Val Leu Asn Pro Ala Ala Ser Leu Pro Thr Leu Ile Trp Asp Ser
        50                  55                  60

ctc ctg gta ccc caa gtg cgg ccg gtt gcc tgg gcc acc ctc ccg ctg      300
Leu Leu Val Pro Gln Val Arg Pro Val Ala Trp Ala Thr Leu Pro Leu
    65                  70                  75

cgg gag agc ccc aag gcc gta gag ctg acc tcg ctg tcc gat gag gac      348
Arg Glu Ser Pro Lys Ala Val Glu Leu Thr Ser Leu Ser Asp Glu Asp
80                  85                  90                  95

agt ggc aaa agc tcc cag ccg ccc agc ccg ccc tcg ccg gcg ccg tcg      396
Ser Gly Lys Ser Ser Gln Pro Pro Ser Pro Pro Ser Pro Ala Pro Ser
                100                 105                 110

tcc ttc tcg tcc acc tcg gcc tcg tcc ctg gag gcc gag gcc ttc atc      444

Ser Phe Ser Ser Thr Ser Ala Ser Ser Leu Glu Ala Glu Ala Phe Ile
            115                 120                 125

gcc ttc cct ggc ttg ggc caa ctt ccc aag cag ctg gcc agg ctc tcg     492
Ala Phe Pro Gly Leu Gly Gln Leu Pro Lys Gln Leu Ala Arg Leu Ser
        130                 135                 140

gtg gcc aag gac ccc cag tcg cgg aag atc ttc aac tgc aaa tat tgt     540
Val Ala Lys Asp Pro Gln Ser Arg Lys Ile Phe Asn Cys Lys Tyr Cys
    145                 150                 155

aac aag gag tac ctc agc ctg ggc gct ctg aag atg cac atc cga agc     588
Asn Lys Glu Tyr Leu Ser Leu Gly Ala Leu Lys Met His Ile Arg Ser
160                 165                 170                 175

cac acg ctg cct tgt gtc tgc acg acc tgt gga aag gcc ttc tct agg     636
His Thr Leu Pro Cys Val Cys Thr Thr Cys Gly Lys Ala Phe Ser Arg
                180                 185                 190

ccc tgg ctg ctt cag ggc cac gtc cgc acc cac act ggt gag aag cca     684
Pro Trp Leu Leu Gln Gly His Val Arg Thr His Thr Gly Glu Lys Pro
            195                 200                 205

ttc tcc tgc tcc cac tgc aac cgt gct ttt gct gac cgc tcc aac ctg     732
Phe Ser Cys Ser His Cys Asn Arg Ala Phe Ala Asp Arg Ser Asn Leu
        210                 215                 220

cgt gcc cac ctc caa acc cac tcg gat gtg aag aga tac cag tgc cag     780
Arg Ala His Leu Gln Thr His Ser Asp Val Lys Arg Tyr Gln Cys Gln
    225                 230                 235

gcc tgt gcc cga acc ttc tcc cgc atg tcc ttg ctc cac aag cac caa     828
Ala Cys Ala Arg Thr Phe Ser Arg Met Ser Leu Leu His Lys His Gln
240                 245                 250                 255

gag tct ggc tgc tcc gga ggc cct cgc tga ccctgctacc tccccatcct     878
Glu Ser Gly Cys Ser Gly Gly Pro Arg
                260

cgctggcatc ttcccggagc tcaccctcct cctcactgcc aggactcctt ccagccttgg   938

tccggggacc tgtggcgtcc atgtctggac ctggttcctg cttggctctc ttggtggcct   998

ttgccgcagg tggctgatgg agtgcctttg tacccgccca gagcctccta cccctcagta 1058

ttcatgaggt gtagcctctg gacacagctg cttcgagcca tagaactaaa gccaacccac 1118

tggctgggaa gcttgaaccc cgctcagggg accccacttc cctacctccc tcaaggaccc 1178

ttcaggccac cttctttgag gtacaacaga ctatgcaata gttcccctcc cccccacccc 1238

gtccagctgt aaccatgcct cagcagggtg gttactggac acatgtccag gtgcccctgg 1298

gcctgggcaa ctgtttcagc cccccgcccc atttgtcctg gtgacacctg tttcacagca 1358

gtttaactgt ctcagaaggg accatgaata atggccatca cttgttaggg gccaagtggg 1418

gtgcttcagc ctggccaatg tgtctcccag aactattttg gggcccaaca ggtggccccg 1478

ggagaaagat gtttacattt taaaggtatt tatattgtaa gcagcatttt gtatagttaa 1538

tatgtacagt ttattgatat tcaataaaat ggttaattta tatactaaaa aaaaaaaaaa 1598

aaaaaaaaaa aaaaa 1613

<210> 2
<211> 264
<212> PRT
<213> Mouse

<400> 2

Met Pro Arg Ser Phe Leu Val Arg Lys Pro Ser Asp Pro Arg Arg Lys
1               5                   10                  15

Pro Asn Tyr Ser Glu Leu Gln Asp Ala Cys Val Glu Phe Thr Phe Gln
            20                  25                  30

Gln Pro Tyr Asp Gln Ala His Leu Leu Ala Ala Ile Pro Pro Pro Glu
        35                  40                  45

Val Leu Asn Pro Ala Ala Ser Leu Pro Thr Leu Ile Trp Asp Ser Leu
    50                  55                  60

Leu Val Pro Gln Val Arg Pro Val Ala Trp Ala Thr Leu Pro Leu Arg
65                  70                  75                  80

Glu Ser Pro Lys Ala Val Glu Leu Thr Ser Leu Ser Asp Glu Asp Ser
                85                  90                  95

Gly Lys Ser Ser Gln Pro Pro Ser Pro Pro Ser Pro Ala Pro Ser Ser
            100                 105                 110

Phe Ser Ser Thr Ser Ala Ser Ser Leu Glu Ala Glu Ala Phe Ile Ala
        115                 120                 125

Phe Pro Gly Leu Gly Gln Leu Pro Lys Gln Leu Ala Arg Leu Ser Val
    130                 135                 140

Ala Lys Asp Pro Gln Ser Arg Lys Ile Phe Asn Cys Lys Tyr Cys Asn
145                 150                 155                 160

Lys Glu Tyr Leu Ser Leu Gly Ala Leu Lys Met His Ile Arg Ser His
                165                 170                 175

Thr Leu Pro Cys Val Cys Thr Thr Cys Gly Lys Ala Phe Ser Arg Pro
            180                 185                 190

Trp Leu Leu Gln Gly His Val Arg Thr His Thr Gly Glu Lys Pro Phe
        195                 200                 205

Ser Cys Ser His Cys Asn Arg Ala Phe Ala Asp Arg Ser Asn Leu Arg
    210                 215                 220

Ala His Leu Gln Thr His Ser Asp Val Lys Arg Tyr Gln Cys Gln Ala
225                 230                 235                 240

Cys Ala Arg Thr Phe Ser Arg Met Ser Leu Leu His Lys His Gln Glu
                245                 250                 255

Ser Gly Cys Ser Gly Gly Pro Arg
            260

<210>  3
<211>  2084
<212>  DNA
<213>  Mouse

<220>
<221>  CDS
<222>  (116)..(925)
<223>  Mouse Snail2 encoding sequence

<400>  3
cagggagccg ggtgacttca gaggcgcctg cctgtccccc gccgcacctg agccaccgcg      60

atgctatagg accgccgcct ggaccgttat ccgcccgcgc ccgcccgcag ccacc atg      118
                                                             Met
                                                             1

ccg cgc tcc ttc ctg gtc aag aaa cat ttc aac gcc tcc aag aag ccc      166
Pro Arg Ser Phe Leu Val Lys Lys His Phe Asn Ala Ser Lys Lys Pro
            5                   10                  15

aac tac agc gaa ctg gac aca cac aca gtt att att tcc cca tat ctc      214
Asn Tyr Ser Glu Leu Asp Thr His Thr Val Ile Ile Ser Pro Tyr Leu
        20                  25                  30

tat gaa agt tac cct ata cct gtc ata cca aaa cca gag atc ctc acc      262
Tyr Glu Ser Tyr Pro Ile Pro Val Ile Pro Lys Pro Glu Ile Leu Thr
    35                  40                  45

tcg gga gca tac agc cct att act gta tgg aca tcg tcg gca gct cca      310
Ser Gly Ala Tyr Ser Pro Ile Thr Val Trp Thr Ser Ser Ala Ala Pro
50                  55                  60                  65

ctc cac tct cct tta ccc agt ggc ctt tct cct ctt act gga tac tcc      358
Leu His Ser Pro Leu Pro Ser Gly Leu Ser Pro Leu Thr Gly Tyr Ser
                70                  75                  80

tca tcc ttg ggg cgt gta agt ccc ccg cct tcc tct gac act tca tcc      406

Ser Ser Leu Gly Arg Val Ser Pro Pro Pro Ser Ser Asp Thr Ser Ser
            85                  90                  95

aag gat cac agt ggt tca gaa agt ccc att agt gac gaa gag gag aga     454
Lys Asp His Ser Gly Ser Glu Ser Pro Ile Ser Asp Glu Glu Glu Arg
        100                 105                 110

ctg cag ccc aag ctt tca gac ccc cat gcc atc gaa gct gag aag ttt     502
Leu Gln Pro Lys Leu Ser Asp Pro His Ala Ile Glu Ala Glu Lys Phe
    115                 120                 125

cag tgc aat tta tgc aat aag acc tat tct acg ttc tct ggg ctg gcc     550
Gln Cys Asn Leu Cys Asn Lys Thr Tyr Ser Thr Phe Ser Gly Leu Ala
130                 135                 140                 145

aaa cac aag cag ctg cac tgt gat gcc cag tct agg aaa tcg ttc agc     598
Lys His Lys Gln Leu His Cys Asp Ala Gln Ser Arg Lys Ser Phe Ser
                150                 155                 160

tgc aag tac tgt gac aag gaa tat gtg agc ctg ggt gcc ctg aag atg     646
Cys Lys Tyr Cys Asp Lys Glu Tyr Val Ser Leu Gly Ala Leu Lys Met
            165                 170                 175

cac att cga acc cac aca ttg cct tgt gtc tgc aag atc tgt ggc aag     694
His Ile Arg Thr His Thr Leu Pro Cys Val Cys Lys Ile Cys Gly Lys
        180                 185                 190

gct ttc tcc aga ccc tgg ctg ctt caa gga cac att aga act cac act     742
Ala Phe Ser Arg Pro Trp Leu Leu Gln Gly His Ile Arg Thr His Thr
    195                 200                 205

ggg gaa aag cct ttc tct tgc cct cac tgc aat agg gct ttt gca gac     790
Gly Glu Lys Pro Phe Ser Cys Pro His Cys Asn Arg Ala Phe Ala Asp
210                 215                 220                 225

aga tca aac ctg agg gca cat ctg cag acc cac tct gat gta aag aaa     838
Arg Ser Asn Leu Arg Ala His Leu Gln Thr His Ser Asp Val Lys Lys
                230                 235                 240

tac cag tgc aaa aac tgc tcc aaa acc ttc tcc aga atg tcg ctt ctg     886
Tyr Gln Cys Lys Asn Cys Ser Lys Thr Phe Ser Arg Met Ser Leu Leu
            245                 250                 255

cat aaa cat gag gag tct ggc tgc tgt gtg gca cac tga gtggcgcaac     935
His Lys His Glu Glu Ser Gly Cys Cys Val Ala His
        260                 265

cagtgtttac tcaaacagaa tgcatttctt cactccaatg acaaatgaca aatgaaagtc   995

caaagacatt ttctcatgtg cttaccaacc aaatagtatg tataaaacca caaaagagtc 1055

acacacacac acacacacac acacacacac acacacacag agagagagag agagagagag 1115

agagagacag acagacagac agacagatac acacacacta cagaacagaa tctatgtact 1175

taaagttaat tcgttctatg tgaagtttaa aattatattt actgacagct agattgaaag 1235

gataaaagat aagaatcttt ctctttaaag atgaagtgaa aagcacattg catcttttct 1295

tactaagaaa gaatacagag atttacactg ctgccaaacc atttcaacca aaggaacagt 1355

atttcttctt aatagaattg taatagtgtt tccaagagga agagagtctg ccagacacta 1415

tctcaggtgc cttataaagt actccaagtt tacttcctta aatgtatgat gcctggttgt 1475

catcagtgaa tgacagcctt ttctggatta cctacaatgt tttaaaacta tattgttaag 1535

agaaaaaaaa ccaaaaacaa gaaaaagaac agaacacaag agaatgtatt aaagtattct 1595

tgttttattt ttgccatgtg tgccttggaa gaggagggaa agacaaactt caaacattcc 1655

tggtgcgtgt cccatgtctt tctttttaaa aaagaatctt aatgttttat aatacaaagt 1715

aatgaaaatg tgcaaaagaa tttcttagac attcagtaat gtacttagac ttttgaaaat 1775

tcatgtgatg gatgcagtaa tacaatgccc ctccaagtgc ctgtcttaat gacttgtgta 1835

gttgatgaac tgatgtaaat ttgtgtttat ttttatacaa ctgaatgaac tctgtatgaa 1895

agtgaggtac ggttaatagc cacgcctata ttcaaccaga atacttgtga aatcaatgtc 1955

cttttttaaa aagtaacttt caaggtctct tttttacaat aaacattttt gagtaaaaaa 2015

aaaaaaaaaa aaaaaaaaaa aaaaaaaaaa aaaaaaaaaa aaaaaaaaaa aaaaaaaaaa 2075

aaaaaaaaa 2084

<210> 4
<211> 269
<212> PRT
<213> Mouse

<400> 4

Met Pro Arg Ser Phe Leu Val Lys Lys His Phe Asn Ala Ser Lys Lys
1               5                   10                  15
Pro Asn Tyr Ser Glu Leu Asp Thr His Thr Val Ile Ile Ser Pro Tyr
            20                  25                  30
Leu Tyr Glu Ser Tyr Pro Ile Pro Val Ile Pro Lys Pro Glu Ile Leu
        35                  40                  45
Thr Ser Gly Ala Tyr Ser Pro Ile Thr Val Trp Thr Ser Ser Ala Ala
    50                  55                  60
Pro Leu His Ser Pro Leu Pro Ser Gly Leu Ser Pro Leu Thr Gly Tyr
65                  70                  75                  80
Ser Ser Ser Leu Gly Arg Val Ser Pro Pro Pro Ser Ser Asp Thr Ser
                85                  90                  95
Ser Lys Asp His Ser Gly Ser Glu Ser Pro Ile Ser Asp Glu Glu Glu
            100                 105                 110
Arg Leu Gln Pro Lys Leu Ser Asp Pro His Ala Ile Glu Ala Glu Lys
        115                 120                 125
Phe Gln Cys Asn Leu Cys Asn Lys Thr Tyr Ser Thr Phe Ser Gly Leu
    130                 135                 140
Ala Lys His Lys Gln Leu His Cys Asp Ala Gln Ser Arg Lys Ser Phe
145                 150                 155                 160
Ser Cys Lys Tyr Cys Asp Lys Glu Tyr Val Ser Leu Gly Ala Leu Lys
                165                 170                 175
Met His Ile Arg Thr His Thr Leu Pro Cys Val Cys Lys Ile Cys Gly
            180                 185                 190
Lys Ala Phe Ser Arg Pro Trp Leu Leu Gln Gly His Ile Arg Thr His

        195                 200                 205
Thr Gly Glu Lys Pro Phe Ser Cys Pro His Cys Asn Arg Ala Phe Ala
    210                 215                 220
Asp Arg Ser Asn Leu Arg Ala His Leu Gln Thr His Ser Asp Val Lys
225                 230                 235                 240
Lys Tyr Gln Cys Lys Asn Cys Ser Lys Thr Phe Ser Arg Met Ser Leu
                245                 250                 255
Leu His Lys His Glu Glu Ser Gly Cys Cys Val Ala His
            260                 265

<210>  5
<211>  1708
<212>  DNA
<213>  Homo sapiens


<220>
<221>  CDS
<222>  (71)..(865)
<223>  Human Snail1 encoding sequence

<400>  5
   ggcacggcct agcgagtggt tcttctgcgc tactgctgcg cgaatcggcg accccagtgc      60
   ctcgaccact atg ccg cgc tct ttc ctc gtc agg aag ccc tcc gac ccc        109
              Met Pro Arg Ser Phe Leu Val Arg Lys Pro Ser Asp Pro
              1               5                   10
   aat cgg aag cct aac tac agc gag ctg cag gac tct aat cca gag ttt       157
   Asn Arg Lys Pro Asn Tyr Ser Glu Leu Gln Asp Ser Asn Pro Glu Phe
       15                  20                  25
   acc ttc cag cag ccc tac gac cag gcc cac ctg ctg gca gcc atc cca       205
   Thr Phe Gln Gln Pro Tyr Asp Gln Ala His Leu Leu Ala Ala Ile Pro
   30                  35                  40                  45
   cct ccg gag atc ctc aac ccc acc gcc tcg ctg cca atg ctc atc tgg       253
   Pro Pro Glu Ile Leu Asn Pro Thr Ala Ser Leu Pro Met Leu Ile Trp
                   50                  55                  60
   gac tct gtc ctg gcg ccc caa gcc cag cca att gcc tgg gcc tcc ctt       301
   Asp Ser Val Leu Ala Pro Gln Ala Gln Pro Ile Ala Trp Ala Ser Leu
               65                  70                  75
   cgg ctc cag gag agt ccc agg gtg gca gag ctg acc tcc ctg tca gat       349
   Arg Leu Gln Glu Ser Pro Arg Val Ala Glu Leu Thr Ser Leu Ser Asp
           80                  85                  90
   gag gac agt ggg aaa ggc tcc cag ccc ccc agc cca ccc tca ccg gct       397
   Glu Asp Ser Gly Lys Gly Ser Gln Pro Pro Ser Pro Pro Ser Pro Ala
       95                  100                 105
   cct tcg tcc ttc tcc tct act tca gtc tct tcc ttg gag gcc gag gcc       445
   Pro Ser Ser Phe Ser Ser Thr Ser Val Ser Ser Leu Glu Ala Glu Ala
   110                 115                 120                 125
   tat gct gcc ttc cca ggc ttg ggc caa gtg ccc aag cag ctg gcc cag       493
   Tyr Ala Ala Phe Pro Gly Leu Gly Gln Val Pro Lys Gln Leu Ala Gln
                   130                 135                 140
   ctc tct gag gcc aag gat ctc cag gct cga aag gcc ttc aac tgc aaa       541
   Leu Ser Glu Ala Lys Asp Leu Gln Ala Arg Lys Ala Phe Asn Cys Lys
   145                 150                 155
   tac tgc aac aag gaa tac ctc agc ctg ggt gcc ctc aag atg cac atc     589
   Tyr Cys Asn Lys Glu Tyr Leu Ser Leu Gly Ala Leu Lys Met His Ile
           160                 165                 170
   cga agc cac acg ctg ccc tgc gtc tgc gga acc tgc ggg aag gcc ttc     637
   Arg Ser His Thr Leu Pro Cys Val Cys Gly Thr Cys Gly Lys Ala Phe
       175                 180                 185
   tct agg ccc tgg ctg cta caa ggc cat gtc cgg acc cac act ggc gag     685
   Ser Arg Pro Trp Leu Leu Gln Gly His Val Arg Thr His Thr Gly Glu
   190                 195                 200                 205
   aag ccc ttc tcc tgt ccc cac tgc agc cgt gcc ttc gct gac cgc tcc     733
   Lys Pro Phe Ser Cys Pro His Cys Ser Arg Ala Phe Ala Asp Arg Ser
                   210                 215                 220
   aac ctg cgg gcc cac ctc cag acc cac tca gat gtc aag aag tac cag     781
   Asn Leu Arg Ala His Leu Gln Thr His Ser Asp Val Lys Lys Tyr Gln
               225                 230                 235
   tgc cag gcg tgt gct cgg acc ttc tcc cga atg tcc ctg ctc cac aag     829
   Cys Gln Ala Cys Ala Arg Thr Phe Ser Arg Met Ser Leu Leu His Lys
           240                 245                 250
   cac caa gag tcc ggc tgc tca gga tgt ccc cgc tga ccctcgaggc          875
   His Gln Glu Ser Gly Cys Ser Gly Cys Pro Arg
       255                 260
   tccctcttcc tctccatacc tgcccctgcc tgacagcctt ccccagctcc agcaggaagg   935
   accccacatc cttctcactg ccatggaatt ccctcctgag tgccccactt ctggccacat   995
   cagccccaca ggactttgat gaagaccatt ttctggttct gtgtcctctg cctgggctct 1055
   ggaagaggcc ttcccatggc catttctgtg gagggagggc agctggcccc cagccctggg 1115
   ggattcctga gctggcctgt ctgcgtgggt tttgtatccc agagctgttt ggatacagct 1175
   gctttgagct acaggacaaa ggctgacaga ctcactggga agctcccacc ccactcaggg 1235
   gaccccactc ccctcacaca caccccccca caaggaaccc tcaggccacc ctccacgagg 1295
   tgtgactaac tatgcaataa tccaccccca ggtgcagccc cagggcctgc ggaggcggtg 1355
   gcagactaga gtctgagatg ccccgagccc aggcagctat ttcagcctcc tgtttggtgg 1415
   ggtggcacct gtttcccggg caatttaaca atgtctgaaa agggactgtg agtaatggct 1475
   gtcacttgtc gggggcccaa gtggggtgct ctggtctgac cgatgtgtct cccagaacta 1535
   ttctgggggc ccgacaggtg ggcctgggag gaagatgttt acatttttaa aggtacactg 1595
   gtatttatat ttcaaacatt ttgtatcaag gaaacgtttt gtatagttat atgtacagtt 1655
   tattgatatt caataaagca gttaatttat atattaaaaa aaaaaaaaaa aaa         1708

<210>  6
<211>  264

<212>  PRT
<213>  Homo sapiens

<400>  6

Met Pro Arg Ser Phe Leu Val Arg Lys Pro Ser Asp Pro Asn Arg Lys
1               5                   10                  15

Pro Asn Tyr Ser Glu Leu Gln Asp Ser Asn Pro Glu Phe Thr Phe Gln
            20                  25                  30

Gln Pro Tyr Asp Gln Ala His Leu Leu Ala Ala Ile Pro Pro Pro Glu
        35                  40                  45

Ile Leu Asn Pro Thr Ala Ser Leu Pro Met Leu Ile Trp Asp Ser Val
    50                  55                  60

Leu Ala Pro Gln Ala Gln Pro Ile Ala Trp Ala Ser Leu Arg Leu Gln
65                  70                  75                  80

Glu Ser Pro Arg Val Ala Glu Leu Thr Ser Leu Ser Asp Glu Asp Ser
                85                  90                  95

Gly Lys Gly Ser Gln Pro Pro Ser Pro Pro Ser Pro Ala Pro Ser Ser
            100                 105                 110

Phe Ser Ser Thr Ser Val Ser Ser Leu Glu Ala Glu Ala Tyr Ala Ala
        115                 120                 125

Phe Pro Gly Leu Gly Gln Val Pro Lys Gln Leu Ala Gln Leu Ser Glu
    130                 135                 140

Ala Lys Asp Leu Gln Ala Arg Lys Ala Phe Asn Cys Lys Tyr Cys Asn
145                 150                 155                 160

Lys Glu Tyr Leu Ser Leu Gly Ala Leu Lys Met His Ile Arg Ser His
                165                 170                 175

Thr Leu Pro Cys Val Cys Gly Thr Cys Gly Lys Ala Phe Ser Arg Pro
            180                 185                 190

Trp Leu Leu Gln Gly His Val Arg Thr His Thr Gly Glu Lys Pro Phe
        195                 200                 205

Ser Cys Pro His Cys Ser Arg Ala Phe Ala Asp Arg Ser Asn Leu Arg
    210                 215                 220

Ala His Leu Gln Thr His Ser Asp Val Lys Lys Tyr Gln Cys Gln Ala
225                 230                 235                 240

Cys Ala Arg Thr Phe Ser Arg Met Ser Leu Leu His Lys His Gln Glu
                245                 250                 255

Ser Gly Cys Ser Gly Cys Pro Arg
            260


<210>  7
<211>  2101
<212>  DNA

<213>  Homo sapiens

<220>
<221>  CDS
<222>  (165)..(971)
<223>  Human Sanil2 enconding sequence

<400>  7
agttcgtaaa ggagccgggt gacttcagag gcgccggccc gtccgtctgc cgcacctgag    60

cacggcccct gcccgagcct ggcccgccgc gatgctgtag ggaccgccgt gtcctcccgc   120

cggaccgtta tccgcgccgg gcgcccgcca gacccgctgg caag atg ccg cgc tcc   176
                                                 Met Pro Arg Ser
                                                 1

ttc ctg gtc aag aag cat ttc aac gcc tcc aaa aag cca aac tac agc     224
Phe Leu Val Lys Lys His Phe Asn Ala Ser Lys Lys Pro Asn Tyr Ser
5                   10                  15                  20

gaa ctg gac aca cat aca gtg att att tcc ccg tat ctc tat gag agt     272
Glu Leu Asp Thr His Thr Val Ile Ile Ser Pro Tyr Leu Tyr Glu Ser
                25                  30                  35

tac tcc atg cct gtc ata cca caa cca gag atc ctc agc tca gga gca     320
Tyr Ser Met Pro Val Ile Pro Gln Pro Glu Ile Leu Ser Ser Gly Ala
            40                  45                  50

tac agc ccc atc act gtg tgg act acc gct gct cca ttc cac gcc cag     368
Tyr Ser Pro Ile Thr Val Trp Thr Thr Ala Ala Pro Phe His Ala Gln
        55                  60                  65

cta ccc aat ggc ctc tct cct ctt tcc gga tac tcc tca tct ttg ggg     416
Leu Pro Asn Gly Leu Ser Pro Leu Ser Gly Tyr Ser Ser Ser Leu Gly
    70                  75                  80

cga gtg agt ccc cct cct cca tct gac acc tcc tcc aag gac cac agt     464
Arg Val Ser Pro Pro Pro Pro Ser Asp Thr Ser Ser Lys Asp His Ser
85                  90                  95                  100

ggc tca gaa agc ccc att agt gat gaa gag gaa aga cta cag tcc aag     512
Gly Ser Glu Ser Pro Ile Ser Asp Glu Glu Glu Arg Leu Gln Ser Lys
                105                 110                 115

ctt tca gac ccc cat gcc att gaa gct gaa aag ttt cag tgc aat tta     560
Leu Ser Asp Pro His Ala Ile Glu Ala Glu Lys Phe Gln Cys Asn Leu
            120                 125                 130

tgc aat aag acc tat tca act ttt tct ggg ctg gcc aaa cat aag cag     608
Cys Asn Lys Thr Tyr Ser Thr Phe Ser Gly Leu Ala Lys His Lys Gln
        135                 140                 145

ctg cac tgc gat gcc cag tct aga aaa tct ttc agc tgt aaa tac tgt     656
Leu His Cys Asp Ala Gln Ser Arg Lys Ser Phe Ser Cys Lys Tyr Cys
    150                 155                 160

gac aag gaa tat gtg agc ctg ggc gcc ctg aag atg cat att cgg acc     704
Asp Lys Glu Tyr Val Ser Leu Gly Ala Leu Lys Met His Ile Arg Thr
165                 170                 175                 180

cac aca tta cct tgt gtt tgc aag atc tgc ggc aag gcg ttt tcc aga 752
His Thr Leu Pro Cys Val Cys Lys Ile Cys Gly Lys Ala Phe Ser Arg
                185                 190                 195

ccc tgg ttg ctt caa gga cac att aga act cac acg ggg gag aag cct 800
Pro Trp Leu Leu Gln Gly His Ile Arg Thr His Thr Gly Glu Lys Pro
            200                 205                 210

ttt tct tgc cct cac tgc aac aga gca ttt gca gac agg tca aat ctg 848
Phe Ser Cys Pro His Cys Asn Arg Ala Phe Ala Asp Arg Ser Asn Leu
        215                 220                 225

agg gct cat ctg cag acc cat tct gat gta aag aaa tac cag tgc aaa 896
Arg Ala His Leu Gln Thr His Ser Asp Val Lys Lys Tyr Gln Cys Lys
    230                 235                 240

aac tgc tcc aaa acc ttc tcc aga atg tct ctc ctg cac aaa cat gag 944
Asn Cys Ser Lys Thr Phe Ser Arg Met Ser Leu Leu His Lys His Glu
245                 250                 255                 260

gaa tct ggc tgc tgt gta gca cac tga gtgacgcaat caatgtttac 991
Glu Ser Gly Cys Cys Val Ala His
                265

tcgaacagaa tgcatttctt cactccgaag ccaaatgaca aataaagtcc aaaggcattt 1051

tctcctgtgc tgaccaacca aataatatgt atagacacac acacatatgc acacacacac 1111

acacacaccc acagagagag agctgcaaga gcatggaatt catgtgttta aagataatcc 1171

tttccatgtg aagtttaaaa ttactatata tttgctgatg gctagattga gagaataaaa 1231

gacagtaacc tttctcttca aagataaaat gaaaagcaca ttgcatcttt tcttcctaaa 1291

aaaatgcaaa gatttacatt gctgccaaat catttcaact gaaaagaaca gtattgcttt 1351

gtaatagagt ctgtaatagg atttcccata ggaagagatc tgccagacgc gaactcaggt 1411

gccttaaaaa gtattccaag tttactccat tacatgtcgg ttgtctggtt gccattgttg 1471

aactaaagcc tttttttgat tacctgtagt gctttaaagt atatttttaa aagggaggaa 1531

aaaaataaca agaacaaaac acaggagaat gtattaaaag tatttttgtt ttgttttgtt 1591

tttgccaatt aacagtatgt gccttggggg aggagggaaa gattagcttt gaacattcct 1651

ggcgcatgct ccattgtctt actattttaa aacattttaa taatttttga aaattaatta 1711

aagatgggaa taagtgcaaa agaggattct tacaaattca ttaatgtact taaactattt 1771

caaatgcata ccacaaatgc aataatacaa taccccttcc aagtgccttt ttaaattgta 1831

tagttgatga gtcaatgtaa atttgtgttt atttttatat gattgaatga gttctgtatg 1891

aaactgagat gttgtctata gctatgtcta taaacaacct gaagacttgt gaaatcaatg 1951

tttctttttt aaaaaacaat tttcaagttt tttttacaat aaacagtttt gatttaaaat 2011

ctcgtttgta tactattttc agagacttta cttgcttcat gattagtacc aaaccactgt 2071

acaaagaatt gtttgttaac aagaaaaaaa                                    2101

<210>  8
<211>  268
<212>  PRT
<213>  Homo sapiens

<400>  8

Met Pro Arg Ser Phe Leu Val Lys Lys His Phe Asn Ala Ser Lys Lys
1               5                   10                  15

Pro Asn Tyr Ser Glu Leu Asp Thr His Thr Val Ile Ile Ser Pro Tyr
            20                  25                  30

Leu Tyr Glu Ser Tyr Ser Met Pro Val Ile Pro Gln Pro Glu Ile Leu
        35                  40                  45

Ser Ser Gly Ala Tyr Ser Pro Ile Thr Val Trp Thr Thr Ala Ala Pro
    50                  55                  60

Phe His Ala Gln Leu Pro Asn Gly Leu Ser Pro Leu Ser Gly Tyr Ser
65                  70                  75                  80

Ser Ser Leu Gly Arg Val Ser Pro Pro Pro Pro Ser Asp Thr Ser Ser
                85                  90                  95

Lys Asp His Ser Gly Ser Glu Ser Pro Ile Ser Asp Glu Glu Glu Arg
            100                 105                 110

Leu Gln Ser Lys Leu Ser Asp Pro His Ala Ile Glu Ala Glu Lys Phe
        115                 120                 125

Gln Cys Asn Leu Cys Asn Lys Thr Tyr Ser Thr Phe Ser Gly Leu Ala
    130                 135                 140

Lys His Lys Gln Leu His Cys Asp Ala Gln Ser Arg Lys Ser Phe Ser
145                 150                 155                 160

Cys Lys Tyr Cys Asp Lys Glu Tyr Val Ser Leu Gly Ala Leu Lys Met
                165                 170                 175

His Ile Arg Thr His Thr Leu Pro Cys Val Cys Lys Ile Cys Gly Lys
            180                 185                 190

Ala Phe Ser Arg Pro Trp Leu Leu Gln Gly His Ile Arg Thr His Thr
        195                 200                 205

Gly Glu Lys Pro Phe Ser Cys Pro His Cys Asn Arg Ala Phe Ala Asp
    210                 215                 220

Arg Ser Asn Leu Arg Ala His Leu Gln Thr His Ser Asp Val Lys Lys
225                 230                 235                 240

Tyr Gln Cys Lys Asn Cys Ser Lys Thr Phe Ser Arg Met Ser Leu Leu
                245                 250                 255

His Lys His Glu Glu Ser Gly Cys Cys Val Ala His

260                 265

<210>  9
<211>  19
<212>  DNA
<213>  Homo sapiens

<400>  9
gatgcacatc cgaagccac
19

<210>  10
<211>  27
<212>  DNA
<213>  Artificial sequence

<220>
<223>  Oligo A

<400>  10
ggtaccatct acacattcac tactaga                                          27

<210>  11
<211>  27
<212>  DNA
<213>  Artificial sequence

<220>
<223>  Oligo B

<400>  11
acgcgttttc caaggacgga aaaagaa                                          27